# EUROPEAN PATENT SPECIFICATION

(11) **EP 1 267 840 B1**
(45) Date of publication and mention of the grant of the patent: **03.06.2009**
(21) Application number: 01938048.4
(22) Date of filing: 26.03.2001
(51) Int. Cl.: A61K 9/16, A61K 9/50, A61K 9/00

(54) **GRANULATED PARTICLES WITH MASKED TASTE**
GESCHMACKMASKIERTE GRANULIERTE TEILCHEN
PARTICULES GRANULEES A MASQUAGE DE GOUT

(30) Priority: 28.03.2000 AT 5242000
(43) Date of publication of application: 02.01.2003
(73) Proprietor: Sandoz AG, 4056 Basel (CH)
(72) Inventor: SCHWARZ, Franz, Xaver, A-6300 Wörgl (AT)
(74) Representative: Wichmann, Hendrik
(86) International application number: PCT/EP2001/003409
(87) International publication number: WO 2001/072284

(56) References cited:
- EP-A- 0 622 083
- WO-A-00/69415
- WO-A-00/76479
- US-A- 4 925 675
- LEHMANN, K. ET AL: "Fast disintegrating controlled release tablets from coated particles" DRUGS MADE GER. (1994), 37(2), 53-60 , XP001016125

## Description

The present invention relates to taste masking granules, e.g. in pharmaceutical compositions comprising at least one pharmaceutically active ingredient wherein a bad taste when taken by patients is reduced or eliminated.

Known ways of masking a bad or bitter taste of a pharmaceutically active ingredient are e.g. described in K. Lehmann et al., Drugs Made in Germany, 37(2), 53-60 (1994): "Fast Disintegrating Controlled Release Tablets from Coated Particles" wherein taste-masking of particles such as paracetamol crystals is obtained by applying a coating containing methacrylic acid and methacrylic ester copolymers. These coated crystals are subsequently compressed to tablets.
EP 0 622 083 A1 describes that taste-masking is obtained by mixing a coated or matrix-based, powdery or granular preparation containing an active substance having an unpleasant taste or odour, e.g. a penem, a cephalosporin antibiotic, or a macrolide antibiotic such as clarithromycin, with a powdery or granular ion exchanger such as cation exchange resins or anion exchange resins.
In WO 00/69415, taste-masking of clarithromycin is obtained by coating clarithromycin alone or admixed with various excipients with a lipid film-forming substance such as stearyl alcohol or stearic acid using a melt granulation process. Fatty alcohols, such as stearyl alcohol, however, are stated to delay the release of the active component from the pharmaceutical formulation.
WO 00/76479 describes tasted-masked granules comprising a bitter tasting drug such as a macrolide, e.g. clarithromycin, which are prepared by dissolving the bitter tasting drug and a combination of two enteric polymers containing a methacrylic acid copolymer and a phthalate polymer in an organic solvent to obtain a taste-masked matrix which may be compacted to granules. When coated, such granules comprising clarithromycin show a release of 84% of the drug within 60 minutes at pH 6.8.
US patent No. 4,925,675 describes taste-masked microencapsulated granules comprising a mixture of erythromycin and a binder as active coatings, protective coatings containing a mixture of polyethylene oxide and polyethylene glycol and an enteric coating which coatings are applied onto a seed. These granules show a dissolution of about 76% - 90% after 30 minutes in Simulated Intestinal Fluid.

Preparation processes for pharmaceutical compositions, wherein a bad taste of an active ingredient (compound) when taken by patients is reduced or eliminated may have disadvantages, e.g. use of organic solvent and/or process steps that are difficult to carry out. Particles of active ingredients have e.g. been coated with film-forming components to mask the taste, which, however, may result in delayed release of the active ingredient. Generally such coating may not dissolve quickly enough in the gastrointestinal tract and in consequence the bioavailability of the active compound may be poor.
We have now found a pharmaceutical composition comprising at least one pharmaceutical active compound wherein a bad taste when taken by patients is reduced or eliminated and from which the pharmaceutical active compound is surprisingly quickly released in the gastrointestinal tract which pharmaceutical composition may be produced simply and, if desired, without the use of organic solvents.

The present invention provides coated pellets of granulated particles wherein pellets consisting essentially of
a) at least one macrolide antibiotic as pharmaceutically active compound, and
b) an organic C₄- C₈ carboxylic acid and/or a surfactant selected from the group of polyethylene glycols, polyoxypropylene-polyoxyethylene condensates, polyethylene glycol fatty acid esters and polyethylene glycol sorbitol fatty acid esters, and/or a hydrocolloid selected from the group of polyvinyl pyrrolidones, starch, cellulose, methyl cellulose and hydroxypropylmethyl cellulose,
are coated with an enteric film-forming composition.

A pharmaceutically active compound, which has a bad taste when taken by patients, includes one or more pharmaceutically active compounds, at least one of which has a bad taste when taken by the patients, preferably macrolide antibiotics, e.g. erythromycins, such as erythromycin A, and compounds derived thereof, such as azithromycin, clarithromycin, roxithromycin, preferably clarithromycin (see e.g. The Merck Index, 12^{th} edition, items 3720, 2400, 946, 8433); and other compounds.
A pharmaceutically active compound or a mixture of pharmaceutically active compounds, of which at least one has a bad taste when taken by the patients, is referred to hereinafter as "active ingredient".
An organic carboxylic acid and/or a surface-active substance and/or a hydrocolloid are referred to hereinafter as "an additive according to the present invention".

An organic carboxylic acid according to the present invention includes one or more organic carboxylic acids, e.g. saturated and unsaturated carboxylic acids; e.g. monocarboxylic acids and polycarboxylic acids, e.g. di- and tricarboxylic acids; e.g. unsubstituted or substituted carboxylic acids, e.g. unsubstituted or substituted by amino, hydroxy, aminocarbonyl, aryl, e.g. phenyl; or carboxymethylcellulose acids, e.g. cellulose, wherein hydroxy groups are substituted, e.g. including carboxymethyl groups. Preferred are (C₄₋₈)carboxylic acids, e.g. unsubstituted or substituted and carboxymethylcellulose acids; more preferred are (C₄₋₈)carboxylic acids. Examples of organic carboxylic acids include e.g. mandelic acid, succinic acid, tartaric acid, fumaric acid, maleic acid, glutaric acid, glutamic acid, citric acid. The weight ratio of an organic carboxylic acid and an active ingredient is not critical and appropriate weight ratios may be found out by pre-testing. Preferred are 0.05 to 5; such as 0.1 to 2; preferably 0.1 to 1; most preferably 0.1 to 0.6. parts of organic carboxylic acid per part of active ingredient.

A surfactant according to the present invention includes one or more surfactants, e.g. substances which may influence the surface forces between other substances, e.g. a wetting agent or an emulsifier, such as polyethylene glycols or polyoxypropylenepolyoxyethylene condensates, e.g. obtainable by condensation of propylene oxide with propylene glycol and condensation of the resulting hydrophobic base with ethylene oxide, e.g. including Pluronics®, e.g. Pluronic F68®; glycerol monostearates, polyethylene glycol fatty acid esters, e.g. Cremophors®; and polyethylene glycol sorbitol fatty acid esters, e.g. Tween® types. The weight ratio of a surfactant and an active ingredient is not critical and appropriate weight ratios may be found out by pre-testing. Preferred are 0.05 to 5; such as 0.1 to 2; preferably 0.1 to 1; most preferably 0.1 to 0.9 parts of the surfactant per part of active ingredient.

A hydrocolloid includes one or more hydrocolloids, e.g. natural and synthetic polymers, which can form colloidal solutions in aqueous systems, for example polyvinyl pyrrolidones, starch, cellulose and cellulose derivatives, e.g. Methocel®, such as methyl cellulose, hydroxypropylmethyl cellulose. In one embodiment, the hydrocolloid includes one or more hydrocolloids selected from the group of polyvinyl pyrrolidone, methyl cellulose and hydroxypropylmethyl cellulose.
The weight ratio of a hydrocolloid and an active ingredient is not critical and appropriate weight ratios may be found out by pre-testing. Preferred are 0.005 to 5; such as 0.005 to 2; preferably 0.005 to 1; most preferably 0.01 to 0.6 parts of a hydrocolloid per part of active ingredient.

Per part of an active ingredient preferably 0.1 to 1.0 parts, e.g. 0.1 to 0.7 parts, such as 0.1, 0.3, 0.5 or 0.6 parts of an additive according to the present invention are present. In one preferred embodiment per part of an active ingredient 0.5 to 0.6 parts of an additive according to the present invention are present.

Pellets preferably comprise in addition to an active ingredient, either
- a surfactant; or
- an organic carboxylic acid and a hydrocolloid; or
- a hydrocolloid; such as two different hydrocolloids; or
- a surfactant and a hydrocolloid; e.g. two surfactants and two hydrocolloids; e.g. more preferably
- a polyoxypropylene-polyoxyethylene condensate; or
- a polyethylene glycol; or
- a polyvinyl pyrrolidone and a hydroxypropylmethyl cellulose; or
- fumaric acid or citric acid and a polyvinyl pyrrolidone;
- a polyoxypropylene-polyoxyethylene condensate, a polyvinylpyrrolidone, a polyethyleneglycol and hydroxypropylmethylcellulose.
The size of the pellets according to the present invention is from 0.2 to 1.0 mm, preferably from 0.2 to 0.5 mm. In pellets there is normally a low amount of fine particles. The pellets are practically free of particles < 0,2 µm.

An enteric film-forming composition comprises an enteric film-forming component, e.g. beside further excipients, e.g. appropriate auxiliaries. An enteric film-forming component according to the present invention includes one or more film-forming components, e.g. a film-forming component is able to form a film around the granulated particles. An enteric film-forming component includes appropriate film-forming components, e.g. according to known film-forming components, e.g. including a known enteric film-forming component, e.g. a film-forming component comprising phthalates, such as cellulose phthalates, e.g. chemically modified cellulose phthalates such as hydroxypropylmethyl cellulose phthalate, cellulose acetate phthalate or poly(meth)acrylates. A standardised coating composition which is soluble in the intestines, e.g. includes poly(meth)acrylates, e.g. Eudragit®, such as Eudragit E®, e.g. Eudragit E 30®. An enteric film-forming composition includes a film-forming composition, which, at a physiological pH has a good solubility in the intestinal tract, and which has no good solubility in water or aqueous liquids of a pH which is administrable to a mammal.

The film-forming composition beside an enteric film-forming component may comprise appropriate excipients, e.g. auxiliaries, e.g. pharmaceutically acceptable excipients according to excipients known to be useful in an enteric coating compositions. An excipient e.g. includes a plasticiser. A plasticiser includes an appropriate plasticiser, e.g. including known plasticisers, e.g. a plasticiser which is based on citric acid or citrates, such as alkyl citrates, e.g. (C₁₋₁₂)alkyl citrates, e.g. including triethyl citrate, acetyl triethyl citrate, acetyl-tri-2-ethylhexyl citrate, and butyl citrates, e.g. tributyl citrate, acetyl tributyl citrate, such as Citroflex®.
Preferably the enteric film-forming composition comprises as an enteric film-forming component a poly(meth)acrylate or a hydroxypropylmethyl cellulose phthalate. Preferably the enteric film-forming composition comprises as an excipient a plasticiser, e.g. ethyl citrate or Citroflex®.
The pellets are coated with such an amount of the film-forming composition that the bad taste of the active ingredient is reduced or eliminated when said pharmaceutical composition is administered to a patient. Coated pellets are obtained.

In another aspect, the present invention provides pellets according to the present invention comprising clarithromycin as the pharmaceutically active compound, e.g. and wherein the coating composition comprises a plasticiser.

Pellets according to the present invention may be produced as appropriate, e.g. according, e.g. analogously, to a method as conventional, e.g. preparing granulated particles which contain an active ingredient, an organic carboxylic acid and/or a surfactant and/or a hydrocolloid; e.g. and pelletizing said granulated particles if the size of the granulated particles is to small and to remove fine particles at least of a size below 0,2 mm; and coating the pellets obtained with an enteric film-forming component; e.g. the coating comprising a plasticiser.
In a preferred embodiment pellets may be produced as follows:
Prior to granulation, particles of an active ingredient and/or of an additive may be processed as appropriate, e.g. ground and/or micronised, or particles of an active ingredient and/or of an additive may be used as obtained from a production process. Dry or wet (moist) granulation of an active ingredient and an additive according to the present invention may be effected as appropriate, e.g. according, e.g. analogously, to a method as conventional.
Dry granulation may be effected e.g. by mixing, milling together, compacting. Wet granulation is effected in the presence of an appropriate granulation liquid, e.g. a granulation liquid according, e.g. analogously, to a conventional granulation liquid. A granulation liquid includes e.g. water, organic solvent or a mixture of water and organic solvent, preferably water. Wet granulation may be effected e.g. by production of a wet (moist) mass comprising an active ingredient, an additive according to the present invention and a granulation liquid and drying. Drying may be carried out as appropriate, e.g. according, e.g. analogously, to a method as conventional. Granulated particles may be obtained, which may be in the form of particles, e.g. including agglomerated/aggregated particles; or pellets, dependent on the production process used. A pelletizing process is a granulation process which enhances the size of particles to obtain pellets as defined above. Such pelletizing may be e.g. achieved under appropriate granulation conditions. Appropriate granulation conditions to obtain pellets may be found by pre-testing.

Pellets as used herein define granulated particles comprising an active ingredient and/or a carboxylic acid and/or a surfactant and/or a hydrocolloid having a size from 0.2 to 1.0 mm, preferably 0.2 to 0.5 mm and having a low part of fine particles, e.g. practically no particles below a size of 0.2 mm.
Particles may e.g. be in the form of powders, grains, granules.

In one preferred embodiment wet granulation to obtain pellets as described herein is effected as follows:
The active ingredient and an additive according to the present invention are premixed and the mixture obtained is processed to a granulatable mixture with a solution of an additive in a granulation liquid in a mixer. The mixture obtained is granulated through a sieve an the granulated particles obtained are dried in a fluidised bed apparatus. The dried granulate obtained is equalised through a 0.5 mm sieve.
In another preferred embodiment the active ingredient is suspended with a solution of additives in a granulation liquid in a high-speed agitator (homogeniser). The suspension obtained is sprayed into a fluidised bed apparatus. Drying conditions in the fluidised bed apparatus equipped with a classifies are chosen such that granulated particles of up to 500 µm are obtained. The classifies is adjusted in that way, that only particles bigger then 200 µm can leave the fluidised bed. The particles obtained may have a size distribution of 200 to 500 µm.

In another preferred embodiment the active ingredient is suspended with a solution of additives according to the present invention in a granulation liquid in a high-speed agitator (homogeniser). In a vacuum mixer, granulation liquid is removed from the resulting suspension. A wet mass is obtained and is granulated through a sieve. The granulated particles obtained are dried in a fluidised bed apparatus. The dried granulate obtained is equalised through a 0.5 mm sieve. Particles below 0.2 mm are removed.
Pellets obtained may be used as such or may be further processed as appropriate, e.g. according, e.g. analogously, to a process as conventional e.g. by breaking up, sieving e.g. fractionated sieving, grinding (milling). Pellets may be obtained in an uniform particle size, e.g. in an appropriate size distribution.
Pellets obtained according to the present invention are coated with an enteric film-forming composition, e.g. in the presence of a plasticiser. Coating may be effected as appropriate, e.g. according, e.g. analogously to a method as conventional in the presence of an appropriate coating liquid, e.g. including a coating liquid according, e.g. analaogously, to a method as conventional, e.g. including water, organic solvent or a mixture of water and organic solvent, preferably water.
In a preferred embodiment coating is effected by sprayed into a fluidised bed apparatus (e.g. Hüttlin HKC 5®) together with an aqueous suspension or dispersion which has the composition indicated in TABLE 2 (figures in grams).

Pellets, comprising an active ingredient and an additive according to the present invention which is coated, e.g. film-coated, by an enteric film-forming composition, are obtained. Coated pellets according to the present invention show surprisingly good bioavailability of the active ingredient, i.e. the active ingredient is released from the pellets despite of the coating practically as quick as from uncoated particles comprising an active ingredient in an environment where desired, e.g. the intestinal tract.

The present invention provides a process for the preparation of coated pellets wherein said pellets consist essentially of
a) at least one macrolide antibiotic as pharmaceutically active compound, and
b) an organic C₄- C₈ carboxylic acid and/or a surfactant selected from the group of polyethylene glycols, polyoxypropylene-polyoxyethylene condensates, polyethylene glycol fatty acid esters and polyethylene glycol sorbitol fatty acid esters, and/or a hydrocolloid selected from the group of polyvinyl pyrrolidones, starch, cellulose, methyl cellulose and hydroxypropylmethyl cellulose,
which process comprises the steps of
a') granulating a macrolide antibiotic as pharmaceutically active compound, an organic C₄- C₈ carboxylic acid and/or a surface-active substance selected from the group of polyethylene glycols, polyoxypropylene-polyoxyethylene condensates, polyethylene glycol fatty acid esters and polyethylene glycol sorbitol fatty acid esters, and/or a hydrocolloid selected from the group of polyvinyl pyrrolidones, starch, cellulose, methyl cellulose and hydroxypropylmethyl cellulose to obtain pellets; and
b') coating pellets obtained in step a') with an enteric film-forming composition.

In one specific embodiment, the hydrocolloid used in step a') is a polyvinyl pyrrolidone, methyl cellulose or hydroxypropylmethyl cellulose.

Coated pellets according to the present invention are useful in the production of pharmaceutical composition.

In another aspect the present invention provides the use of coated pellets according to the present invention in the production of pharmaceutical compositions.

The coated pellets, may be present in the pharmaceutical composition according to the present invention as such or, preferably, in mixture with appropriate excipients/auxiliaries. Appropriate excipients/auxiliaries in pharmaceutical composition according to the present invention include pharmaceutically acceptable excipients/auxiliaries according, e.g. analogously, to conventional excipients/auxiliaries in pharmaceutical compositions. Preferably a pharmaceutical composition according to the present invention comprises excipients/auxiliaries.

In another aspect the present invention provides a pharmaceutical composition, e.g. for oral administration, comprising pellets according to the present invention in combination with pharmaceutically acceptable excipients/auxiliaries.

A pharmaceutical composition according to the present invention is administered as appropriate, e.g. orally. Pharmeuctical compositions according to the present invention may be in an appropriate form, e.g. the form of granules, grains, powders or pellets; or in the form of (coated) tablets. Pharmaceutical compositions in the form of (coated) tablets may be obtained as appropriate, e.g. according, e.g. analogously, to a method as conventional, e.g. by compressing coated granulated particles according to the present invention, e.g. as such, or e.g. mixed with appropriate tabletting excipients, to obtain tablets; and optionally coating tablets thus obtained. Appropriate tabletting excipients include tabletting excipients according, e.g. analogously, to conventional tabletting excipients.

Alternatively, pharmaceutical composition is in the form of a tablet, e.g. coated tablet.

We have found that granulated particles according to the present invention are e.g. useful for the production of an, e.g. dry, powder for oral administration (syrup granulate).

In another aspect the present invention provides a dry powder for oral administration comprising pellets according to the present invention beside pharmaceutically acceptable excipients/auxiliaries.

In another aspect the present invention provides a pharmaceutical composition according to the present invention in the form of a dry powder for oral administration.

A dry powder may be in the form of a powder comprising coated pellets according to the present invention in mixture with one or more excipients, e.g. auxiliaries. A dry powder may be obtained as appropriate, e.g. according, e.g. analogously, to a method as conventional, and is preferably obtained as follows:
Coated pellets comprising an active ingredient, in an appropriate form, e.g. in the form of granules, grains, powders; may be mixed with one or more appropriate pharmaceutically acceptable excipients, e.g. auxiliaries, e.g useful in the production of a dry powder for oral administration. Mixing may be carried out e.g. according to a method as conventional. A mixture obtained, e.g. a final powder/grain/granule mixture, or an intermediate powder/grain/granule mixture obtained, may be further processed, e.g. granulated, compacted, broken, milled, sieved as appropriate. A dry powder for oral administration may be obtained, e.g. wherein the particles have a desired, e.g. uniform, particle size, e.g. an appropriate size distribution.
Pharmaceutically acceptable excipients which are useful in the production of a dry powder for oral administration include e.g.
- sugars, e.g. chemically modified, e.g. including fructose, glucose, saccharose, sugar alcohols, e.g. chemically modified,
- sweeteners, e.g. nutritive and artificial, e.g. Na-saccharin, including aspartam;
- flow promoters, e.g. including silicium dioxodes, e.g. colloidal, such as aerosils®;
- thickener, e.g. guar flour, xantham gum;methylcellose,
- binder, e.g. polyvinylpyrrolidones, celluloses;
- flavoring agents, such as organic acids, e.g. citric acid, NaCl, natural and artifical flavors;
- preservatives, such as potassium sorbate, sodiumbenzoate,;
- dyestuffs (colourants) such as TiO₂;
- surfactants;
preferably sugars and/or sweeteners and/or fillers and/or thickeners, and/or preservatives and/or dyestuffs and/or flavoring agents.

A dry powder may be provided in a pharmaceutical dosage form, e.g. in a container, e.g. sachet, bottle.

In another aspect a pharmaceutical dosage form is provided, comprising a dry powder according to the present invention in a container, e.g. a bottle, sachet, e.g. containing an active ingredient corresponding to a desired amount, e.g. per dosage form.

A dry powder according to the present invention may be administered as such or in the form of a syrup, e.g. in the form of a suspension or emulsion. A dry powder according to the present invention may be reconstituted by adding a liquid, e.g. an aqueous liquid, preferably water, to obtain a syrup, e.g. in the form of a suspension or emulsion, e.g. a syrup which is pharmaceutically administrable.

In another aspect, the present invention provides a pharmaceutical suspension, e.g. emulsion, e.g. syrup, which is reconstituted by adding a liquid to a dry powder according to the present invention.

A syrup produced according to the present invention has a good taste, which remains unchanged for at least one week. Dissolution of the active ingredient at pH 6.8 is quick, which means good bioavailability, i.e. the active ingredient is released in sufficient quantity in the gastrointestinal tract.

### Examples

The following examples illustrate the present invention.
In all of the examples, the pharmaceutically active compound (active ingredient) is clarithromycin.
The following abbreviations are used in the examples:
HMPT: hydroxypropylmethyl cellulose
PVP: polyvinyl pyrrolidone, e.g. Kollidon 25®
Pluronic: polyoxypropylene-polyoxyethylene condensate, e.g. Pluronic®, such as Pluronic F68®
PEG: polyethylene glycol, e.g. polyethylene glycol 6000®
Eudragit: film-forming component based on acrylate, e.g. Eudragit®, such as Eudragit L30 D 55®
HMPT-PHT: hydroxypropylmethyl cellulose acetate phthalate, e.g. 30% dispersion in water Citroflex: plasticiser based on citric acid or citrates, e.g. Citroflex®

### A. Preparation of granulated particles

1000 g of the active ingredient and quantities of an additive (in grams) as indicated in TABLE 1

**TABLE 1**

| **Additive** | **Example 1:** | **Example 2:** | **Example 3:** | **Example 4:** | **Example 5:** | **Example 6:** |
|---|---|---|---|---|---|---|
| HMPT: | 280 | - | - | - | - | - |
| PVP | 14 | 14 | 14 | - | - | - |
| fumaric acid | - | 117 | - | - | - | - |
| citric acid | - | - | 280 | - | - | - |
| Pluronic | - | - | - | 500 | - | 500 |
| PEG | - | - | - | - | 500 | - |

are granulated as set out in Examples 1 to 6.

### Examples 1 and 2

The active ingredient is premixed whilst dry with HMPT or fumaric acid and processed to a granulatable mixture with a solution of PVP in 280 g of water in a mixer (e.g. Stephan mixer). The mixture obtained is granulated through a sieve. The granulated particles obtained are dried in a fluidised bed apparatus (e.g. Glatt WSG 5). The dried granulate obtained is equalised through a 0.5 mm sieve. Particles below 0.2 mm are removed.

### Example 3

The active ingredient is suspended with a solution of the citric acid and the PVP in 2000 ml of water in a high-speed agitator (homogeniser, e.g. Ultra Turrax). The suspension obtained is sprayed into a fluidised bed apparatus. Drying conditions in the fluidised bed apparatus equipped with a classifier are chosen such that granulated particles of up to 500 µm are obtained. The classifier is adjustet in that way, that only particels bigger then 200 µm can leave the fluidised bed. So the granulated particles (pellets) obtained have a distribution between 200 and 500 µm.

### Examples 4 and 5

The active ingredient is suspended with a solution of the Pluronic or with a solution of the PVP in 7000 ml of water in a high-speed agitator (homogeniser, e.g. Ultra Turrax). In a vacuum mixer, water is removed from the resulting suspension. A wet mass is obtained and granulated through a sieve. The granulated particles obtained are dried in a fluidised bed apparatus (e.g. Glatt WSG 5). The dried granulate obtained is equalised through a 0.5 mm sieve. Particles below 0.2 mm are removed.

### Example 6

The active ingredient is suspended with a solution of the Pluronic in 7000 ml of water in a high-speed agitator (homogeniser, e.g. Ultra Turrax). The suspension obtained is sprayed into a fluidised bed apparatus. Drying conditions in the fluidised bed apparatus equipped with a classifier are chosen such that granulated particles of up to 500 µm are obtained. The classifier is adjustet in that way, that only particles bigger then 200 µm can leave the fluidised bed. So the particles obtained have a distribution between 200 and 500 µm.

According to examples A1 to A6_pellets, i.e. granulated particles of a size of 0.2 to 0.5 mm are obtained, practically free of particles having a size of below 0.2 mm.

### B. Preparation of coated, granulated particles

1000 g of granulated particles (pellets) obtained according to examples A1 to A6 having a particle size of up to 0.5 mm and from which particles below 200 µm are separated off are sprayed into a fluidised bed apparatus (e.g. Hüttlin HKC 5®) together with an aqueous suspension or dispersion which has the composition indicated in TABLE 2 (figures in grams)

**TABLE 2**

| **Coating** | **Example 1:** | **Example 2:** | **Example 3:** | **Example 4:** | **Example 5:** | **Example 6:** |
|---|---|---|---|---|---|---|
| Eudragit | 2670 | - | 2670 | 2670 | - | 2670 |
| HMPT-PHT: | - | 2667 | - | - | 2667 | - |
| triethyl citrate | - | 200 | - | - | 200 | - |
| Citroflex | 160 | - | 160 | 160 | - | 160 |
| water | 2500 | 3800 | 2500 | 2500 | 3800 | 2500 |

in such a way that practically no agglomeration of the particles takes place and the particles are provided with an enteric coating. Coated pellets are obtained having a particle size which practically corresponds to the size of the particles obtained according to Examples A1 to A6 apart from the thickness of the film from which the particles are surrounded.

### C. Preparation of dry powders and syrups

30.41 g of saccharose, 0.3 g of silicon dioxide, e.g. Aerosil®, such as Aerosil 200®, 0.09 g of xanthan gum, 0.04 g of citric acid, 0.15 g of NaCl, 0.12 g of titanium dioxide, 0.24 g of potassium sorbate, 0.10 g of Na saccharin and 0.90 g of an aromatic as a preservative are mixed whilst dry with 7.65 g of coated particles obtained according to examples A and B. 40 g of a homogeneous dry powder is obtained.

A dry powder obtained is filled into dosage forms containing e.g. 12 doses of 250 mg clarithromycin, e.g. to prepare a 60 ml suspension, by reconstituting the dry powder in a liquid, e.g. water.

Syrups (pharmaceutical suspensions) obtained are pharmaceutically administrable, have a pleasant taste and are not bitter. In contrast, syrups made from dry powders, in which clarithromycin is not in the form of granulated coated particles, but otherwise contain the same excipients, have a bad and bitter taste.
The release of clarithromycin from syrups obtained is practically complete within ca. 15 minutes at a pH of 6.8. This corresponds to good bioavailability of the clarithromycin. In contrast, from syrups prepared from dry powders, in which the active ingredient is not present in the form of granulated coated particles, but otherwise contain the same excipients, clarithromycin is released slowly at a pH of 6.8, e.g. after 15 minutes less than 20% of the clarithromycin has been released, and after ca. one hour less than 40%. This does not correspond to good bioavailability of the clarithromycin.

## Claims

1. Coated pellets of granulated particles, which pellets are **characterised in that** a bad taste of a pharmaceutically active compound when taken by patients is reduced or eliminated, wherein said pellets consisting essentially of
a) at least one macrolide antibiotic as pharmaceutically active compound, and
b) an organic C₄- C₈ carboxylic acid and/or a surfactant selected from the group of polyethylene glycols, polyoxypropylene-polyoxyethylene condensates, polyethylene glycol fatty acid esters and polyethylene glycol sorbitol fatty acid esters, and/or a hydrocolloid selected from the group of polyvinyl pyrrolidones, starch, cellulose, methyl cellulose and hydroxypropylmethyl cellulose,
are coated with an enteric film-forming composition.

2. The coated pellets of claim 1 wherein clarithromycin is the pharmaceutically active compound.

3. The coated pellets of claim 1 wherein the pellets have a size of from 0.2 mm to 1.0 mm, preferably of from 0.2 mm to 0.5 mm.

4. The coated pellets of claim 1 wherein the organic C₄- C₈ carboxylic acid is mandelic acid, succinic acid, tartaric acid, fumaric acid, maleic acid, glutaric acid, glutamic acid or citric acid.

5. The coated pellets of claim 1 wherein the surfactant is a polyethylene glycol or a polyoxypropylene-polyoxyethylene condensate.

6. The coated pellets of claim 1, wherein the hydrocolloid is a polyvinyl pyrrolidone, methyl cellulose or hydroxypropylmethyl cellulose.

7. The coated pellets of claim 1 wherein the hydrocolloid is a polyvinyl pyrrolidone and hydroxypropylmethyl cellulose.

8. The coated pellets of claim 1 wherein the organic C₄- C₈ carboxylic acid is fumaric acid or citric acid and the hydrocolloid is a polyvinyl pyrrolidone.

9. The coated pellets of claim 1 wherein the surfactant is a polyethylene glycol and a polyoxypropylene- polyoxyethylene condensate and the hydrocolloid is a polyvinyl pyrrolidone and hydroxypropylmethyl cellulose.

10. Use of coated pellets according to any one of claims 1 to 9 in the production of pharmaceutical compositions.

11. A pharmaceutical composition comprising coated pellets according to any one of claims 1 to 9 in combination with pharmaceutically acceptable excipients/auxiliaries.

12. The pharmaceutical composition of claim 11 in the form of a dry powder for oral administration.

13. The pharmaceutical composition of claim 11 in the form of a suspension.

14. A process for the preparation of coated pellets according to claim 1 which process comprises the steps of
a) granulating a macrolide antibiotic as pharmaceutically active compound, an organic C₄ - C₈ carboxylic acid and/or a surface-active substance selected from the group of polyethylene glycols, polyoxypropylene-polyoxyethylene condensates, polyethylene glycol fatty acid esters and polyethylene glycol sorbitol fatty acid esters, and/or a hydrocolloid selected from the group of polyvinyl pyrrolidones, starch, cellulose, methyl cellulose and hydroxypropylmethyl cellulose to obtain pellets; and
b) coating pellets obtained in step a) with an enteric film-forming composition.

15. The process of claim 14 wherein the hydrocolloid is a polyvinyl pyrrolidone, methyl cellulose or hydroxypropylmethyl cellulose.

## Patentansprüche

1. Beschichtete Pellets granulierter Partikel, wobei die Pellets **dadurch gekennzeichnet sind, dass** ein schlechter Geschmack einer pharmazeutisch aktiven Verbindung, wenn von Patienten eingenommen, reduziert oder eliminiert ist, wobei die Pellets, die im Wesentlichen bestehen aus
a) mindestens einem Makrolidantibiotikum als pharmazeutisch aktive Verbindung und
b) einer organischen C₄-C₈-Carbonsäure und/oder einem oberflächenaktiven Mittel, ausgewählt aus der Gruppe aus Polyethylenglykolen, Polyoxypropylen-Polyoxyethylen-Kondensaten, Polyethylenglykolfettsäureestern und Polyethylenglykolsorbitolfettsäureestern, und/oder einem Hydrokolloid, ausgewählt aus der Gruppe von Polyvinylpyrrolidonen, Stärke, Cellulose, Methylcellulose und Hydroxypropylmethylcellulose,
mit einer enterischen Film bildenden Zusammensetzung beschichtet sind.

2. Beschichtete Pellets nach Anspruch 1, wobei Clarithromycin die pharmazeutisch aktive Verbindung ist.

3. Beschichtete Pellets nach Anspruch 1, wobei die Pellets eine Größe von 0,2 mm bis 1,00 mm, vorzugsweise von 0,2 mm bis 0,5 mm haben.

4. Beschichtete Pellets nach Anspruch 1, wobei die organische C₄-C₈-Carbonsäure Mandelsäure, Bernsteinsäure, Weinsäure, Fumarsäure, Maleinsäure, Glutarsäure, Glutaminsäure oder Citronensäure ist.

5. Beschichtete Pellets nach Anspruch 1, wobei das oberflächenaktive Mittel ein Polyethylenglykol oder ein Polyoxypropylen-Polyoxyethylen-Kondensat ist.

6. Beschichtete Pellets nach Anspruch 1, wobei das Hydrokolloid ein Polyvinylpyrrolidon, Methylcellulose oder Hydroxypropylmethylcellulose ist.

7. Beschichtete Pellets nach Anspruch 1, wobei das Hydrokolloid ein Polyvinylpyrrolidon und Hydroxypropylmethylcellulose ist.

8. Beschichtete Pellets nach Anspruch 1, wobei die organische C₄-C₈-Carbonsäure Fumarsäure oder Citronensäure ist, und das Hydrokolloid ein Polyvinylpyrrolidon ist.

9. Beschichtete Pellets nach Anspruch 1, wobei das oberflächenaktive Mittel ein Polyethylenglykol und ein Polyoxypropylen-Polyoxyethylen-Kondensat ist, und das Hydrokolloid ein Polyvinylpyrrolidon und Hydroxypropylmethylcellulose ist.

10. Verwendung beschichteter Pellets gemäß einem beliebigen der Ansprüche 1 bis 9 bei der Herstellung pharmazeutischer Zusammensetzungen.

11. Pharmazeutische Zusammensetzung, umfassend beschichtete Pellets gemäß einem beliebigen der Ansprüche 1 bis 9 in Kombination mit pharmazeutisch verträglichen Exzipientien/ Hilfsmitteln.

12. Pharmazeutische Zusammensetzung nach Anspruch 11 in der Form eines trockenen Pulvers zur oralen Verabreichung.

13. Pharmazeutische Zusammensetzung nach Anspruch 11 in der Form einer Suspension.

14. Verfahren zur Herstellung beschichteter Pellets gemäß Anspruch 1, wobei das Verfahren die folgenden Schritte umfasst:
a) Granulieren eines Makrolidantibiotikums als pharmazeutisch aktive Verbindung, einer organischen C₄-C₈-Carbonsäure und/oder einer oberflächenaktiven Substanz, ausgewählt aus der Gruppe von Polyethylenglykolen, Polyoxypropylen-Polyoxyethylen-Kondensaten, Polyethylenglykolfettsäureestern und Polyethylenglykolsorbitolfettsäureestern, und/oder eines Hydrokolloids, ausgewählt aus der Gruppe von Polyvinylpyrrolidonen, Stärke, Cellulose, Methylcellulose und Hydroxypropylmethylcellulose, um Pellets zu erhalten; und
b) Beschichten der in Schritt a) erhaltenen Pellets mit einer enterischen Film bildenden Zusammensetzung.

15. Verfahren nach Anspruch 14, wobei das Hydrokolloid ein Polyvinylpyrrolidon, Methylcellulose oder Hydroxypropylmethylcellulose ist.

## Revendications

1. Grains enrobés comprenant des particules granulées, lesdits grains, qui consistent essentiellement en :
a) au moins un antibiotique de la famille des macrolides en tant que composé pharmaceutiquement actif, et
b) un acide carboxylique organique en C₄-C₈ et/ou un agent tensioactif choisi dans le groupe constitué par les polyéthylène glycols, les condensats de polyoxypropylène-polyoxyéthylène, les esters d'acides gras de polyéthylène glycol et les esters d'acides gras de polyéthylène glycol sorbitol, et/ou un hydrocolloïde choisi dans le groupe constitué par les polyvinylpyrrolidones, l'amidon, la cellulose, la méthylcellulose et l'hydroxypropylméthylcellulose,
étant recouverts d'une composition filmogène à délitage entérique.

2. Grains enrobés selon la revendication 1, dans lesquels la clarithromycine est le composé pharmaceutiquement actif.

3. Grains enrobés selon la revendication 1, lesquels ont une taille de 0,2 mm à 1,0 mm, de préférence de 0,2 mm à 0,5 mm.

4. Grains enrobés selon la revendication 1, dans lesquels l'acide carboxylique organique en C₄-C₈ est l'acide mandélique, l'acide succinique, l'acide tartrique, l'acide fumarique, l'acide maléique, l'acide glutarique, l'acide glutamique ou l'acide citrique.

5. Grains enrobés selon la revendication 1, dans lesquels l'agent tensioactif est un polyéthylène glycol ou un condensat de polyoxypropylène-polyoxyéthylène.

6. Grains enrobés selon la revendication 1, dans lesquels l'hydrocolloïde est une polyvinylpyrrolidone, la méthylcellulose ou l'hydroxypropylméthylcellulose.

7. Grains enrobés selon la revendication 1, dans lesquels l'hydrocolloïde est une polyvinylpyrrolidone ou l'hydroxypropylméthylcellulose.

8. Grains enrobés selon la revendication 1, dans lesquels l'acide carboxylique organique en C₄-C₈ est l'acide fumarique ou l'acide citrique et l'hydrocolloïde est une polyvinylpyrrolidone.

9. Grains enrobés selon la revendication 1, dans lesquels l'agent tensioactif est un polyéthylène glycol ou un condensat de polyoxypropylène-polyoxyéthylène et l'hydrocolloïde est une polyvinylpyrrolidone ou l'hydroxypropylméthylcellulose.

10. Utilisation de grains enrobés selon l'une quelconque des revendications 1 à 9, dans la production de compositions pharmaceutiques.

11. Composition pharmaceutique comprenant des grains enrobés selon l'une quelconque des revendications 1 à 9, en combinaison avec des excipients/auxiliaires pharmaceutiquement acceptables.

12. Composition pharmaceutique selon la revendication 11, sous forme de poudre sèche destinée à une administration par voie orale.

13. Composition pharmaceutique selon la revendication 11, sous forme de suspension.

14. Procédé de préparation de grains enrobés selon la revendication 1, lequel comprend les étapes consistant à :
a) granuler un antibiotique de la famille des macrolides en tant que composé pharmaceutiquement actif, un acide carboxylique organique en C₄-C₈ et/ou une substance tensioactive choisie dans le groupe constitué par les polyéthylène glycols, les condensats de polyoxypropylène-polyoxyéthylène, les esters d'acides gras de polyéthylène glycol et les esters d'acides gras de polyéthylène glycol sorbitol, et/ou un hydrocolloïde choisi dans le groupe constitué par les polyvinylpyrrolidones, l'amidon, la cellulose, la méthylcellulose et l'hydroxypropylméthylcellulose, afin d'obtenir des grains ; et
b) recouvrir les grains obtenus à l'étape a) d'une composition filmogène à délitage entérique.

15. Procédé selon la revendication 14, dans lequel l'hydrocolloïde est une polyvinylpyrrolidone, la méthylcellulose ou l'hydroxypropylméthylcellulose.
